(19) Europäisches Patentamt
European Patent Office
Office européen des brevets

(11) EP 0 815 845 B1

(12) **FASCICULE DE BREVET EUROPEEN**

(45) Date de publication et mention de la délivrance du brevet:
**26.01.2000 Bulletin 2000/04**

(51) Int Cl.[7]: **A61K 7/48**, A61K 7/06, A61K 7/16

(21) Numéro de dépôt: **97401255.1**

(22) Date de dépôt: **04.06.1997**

(54) **Composition cosmétique et/ou dermatologique acide contenant un poly(acide 2-acrylamido 2-méthylpropane sulfonique) réticulé et neutralisé à au moins 90 %**

Kosmetische und/oder dermatalogische säure Zusammensetzung, die eine vernetzte und mindestens 90% neutralisierte Poly-2-acrylamide-2-methylpropane Sulfonsäure enthält

Cosmetic and/or dermatological acidic composition containing a crosslinked and at least 90% neutralized polymer of 2-acrylamide-2-methylpropane sulfonic acid

(84) Etats contractants désignés:
**AT BE CH DE ES FR GB IT LI NL SE**

(30) Priorité: **28.06.1996 FR 9608108**

(43) Date de publication de la demande:
**07.01.1998 Bulletin 1998/02**

(73) Titulaire: **L'OREAL**
**75008 Paris (FR)**

(72) Inventeurs:
- **Dupuis, Christine**
  **75008 Paris (FR)**
- **Sebillotte-Arnaud, Laurence**
  **94240 L'Hay Les Roses (FR)**
- **Hansenne, Isabelle**
  **75017 Paris (FR)**
- **Lorant, Raluca**
  **94320 Thiais (FR)**
- **Maubru, Mireille**
  **78400 Chatou (FR)**

(74) Mandataire: **Miszputen, Laurent**
**L'OREAL-DPI**
**6 rue Bertrand Sincholle**
**92585 Clichy Cédex (FR)**

(56) Documents cités:
**EP-A- 0 642 781**
**EP-A- 0 680 748**
**US-A- 3 931 089**
**US-A- 5 114 706**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 815 845 B1

Printed by Jouve, 75001 PARIS (FR)

## Description

L'invention se rapporte à une composition cosmétique et/ou dermatologique contenant un milieu aqueux acide ayant un pH inférieur à 5 et au moins un polymère poly(acide 2-acrylamido 2-méthyl-propane sulfonique) réticulé et neutralisé à au moins 90%

L'invention se rapporte aussi à une utilisation de cette composition pour le traitement cosmétique des matières kératiniques en particulier la peau, les cheveux et les muqueuses notamment à un procédé non-thérapeutique de dépigmentation de la peau.

A différentes périodes de leur vie, certaines personnes voient apparaître sur la peau et plus spécialement sur les mains des tâches plus foncées et/ou plus colorées, conférant à la peau une hétérogénéité. En général, ces tâches sont dues à une production importante de mélanine dans l'épiderme et/ou le derme de la peau.

Ces tâches peuvent être liées à plusieurs phénomènes et plus spécialement au vieillissement. Dans certains cas, ces tâches peuvent devenir cancéreuses. Aussi, on cherche de plus en plus à diminuer, voire éliminer, ces tâches. Pour traiter ces tâches, on utilise des actifs organiques comme l'acide kojique, l'acide caféique, l'acide salicylique et ses dérivés.

On traite également les cheveux abîmés, par des compositions à base d'actifs acides pour les tonifier, leur redonner de la vigueur, renforcer les fibres kératiniques.

Les compositions classiquement utilisées dans les domaines cosmétique et/ou dermatologique sont des émulsions eau-dans-huile (E/H), des émulsions huile-dans-eau (H/E), ou des gels aqueux, dans lesquels il est souvent difficile, voire même impossible d'incorporer des actifs acides organiques comme l'acide kojique, l'acide caféique, l'acide salicylique et ses dérivés.

En général, ces actifs acides ont tendance à recristalliser ou à se dégrader. Il s'ensuit une perte d'efficacité plus ou moins importante de ces compositions, selon le degré de recristallisation et/ou de dégradation, ce qui va à l'encontre de l'objectif recherché. En outre, cette recristallisation ou dégradation peut modifier la stabilité globale de ces compositions ainsi que leur aspect, ce qui peut détourner l'utilisateur de ces compositions à traitement spécifique.

Pour solubiliser certains de ces actifs, il est connu d'utiliser des émulsions E/H ou H/E dans lesquelles la phase aqueuse présente un pH acide. Pour que ces émulsions soient stables (non séparation des phases aqueuse et huileuse), il est nécessaire d'utiliser des émulsionnants (ou tensioactifs). Malheureusement, ces tensioactifs sont souvent irritants pour la peau. En outre ces émulsions manquent souvent de fraîcheur à l'application, ce qui peut gêner leurs utilisations pendant les périodes chaudes de l'année et/ou dans les pays chauds. Un gel aqueux est beaucoup plus apprécié dans ces conditions d'utilisation. Mais sa trop grande quantité d'eau ne permet pas d'y introduire les actifs présentant un certain caractère lipophile. La stabilité de ces gels est par ailleurs médiocre.

Il subsiste donc le besoin d'une composition stable ayant l'aspect d'un gel, utilisable notamment dans les domaines cosmétique et/ou dermatologique permettant une solubilisation suffisante des actifs acides généralement utilisés dans ces domaines en vue d'une efficacité maximum.

On a déjà envisagé dans la demande EP-A-680 748 d'utiliser dans cet objectif des compositions acides sous forme de gel contenant des copolymères ou des homopolyméres cationiques réticulés, substantiellement solubles dans les milieux aqueux et notamment dans l'eau et constitués de motifs résultant de la réaction entre (i) un monomère cationique à insaturation éthylénique ou un mélange cationique de monomères à insaturation éthylénique et (ii) un agent de réticulation à polyinsaturation éthylénique. Ces gélifiants permettent de stabiliser et de solubiliser ces actifs acides dans des compositions contenant dans des milieux aqueux riches en solvant organique.

On a également déjà envisagé dans la demande EP-A-642 781 d'utiliser comme gélifiants et stabilisants, un copolymère anionique réticulé substantiellement soluble dans l'eau et constitué de motifs dérivant de la réaction entre (i) l'acrylamide, (ii) l'acide 2-acrylamido 2-méthyl propane sulfonique et (iii) au moins un composé à polyinsaturation oléfinique (agent de réticulation), dans des émulsions huile-dans-eau acides contenant des actifs organiques acides.

Ces deux familles de polymère présentent l'inconvénient de ne pas permettre la réalisation de gels spontanément transparents, ce qui est gênant pour l'aspect esthétique du produit final. De plus, en présence de certains actifs organiques acides comme l'acide glycolique, ces polymères perdent leur pouvoir épaississant et/ou gélifiant et ne permettent pas d'obtenir des formulations de viscosité élevée et stable.

Le document US5114706 a trait à un shampooing conditionneur comprenant une émulsion aqueuse, un tensio-actif anionique détergent, un conditionneur cationique, un alcool gras, une huile de silicone et un polymère anionique réticulé qui est par exemple un poly(acide 2-acrylamido 2-metgylpropane sulfonique) réticulé. Ce polymère n'est pas à la fois réticulé à au moins 90% et dans une composition dont la phase aqueuse a un pH inférieur à 5.

La demanderesse a découvert de manière surprenante une nouvelle famille de polymères épaississants et/ou gélifiants permettant d'obtenir des formulations cosmétiques et dermatologiques acides sous forme de gels transparents, homogènes et pouvant atteindre des viscosités élevées et stables dans le temps à température ambiante ou à des températures plus élevées.

Ils permettent de plus de solubiliser et de stabiliser les actifs organiques acides dans une composition cosmétique ou dermatologique contenant un milieu aqueux acide à des pH inférieurs ou égal à 5.

En particulier, ils permettent de solubiliser et de stabiliser les actifs acides organiques dans une composition cosmétique ou dermatologique contenant un milieu aqueux acide riche en solvant organique.

On entend par composition contenant un milieu aqueux riche en solvant organique par composition contenant au moins 45% en poids de solvant organique par rapport au poids total de la composition.

Enfin, ils permettent de réaliser des gels transparents, non-coulants, non-filants, doux et glissants à l'application.

Ainsi, l'invention a pour objet une composition cosmétique et/ou dermatologique contenant un milieu aqueux acide cosmétiquement acceptable et au moins un poly(acide 2-acrylamido 2-méthylpropane sulfonique) réticulé et neutralisé à au moins 90%.

La composition de l'invention a une certaine consistance et/ou tenue ; elle n'est pas filante, c'est-à-dire qu'elle ne forme pas de fil lorsqu'on la prend au doigt. Elle se présente plus spécialement sous forme d'un gel.

De façon avantageuse, le pH du milieu aqueux est choisi dans la gamme allant de 1 à 4. Au delà de la valeur de pH 6, la formulation ne présente plus de difficultés.

Les polymères poly(acide 2-acrylamido 2-méthylpropane sulfonique) réticulés et pratiquement ou totalement neutralisés, conformes à l'invention, sont hydrosolubles ou gonflables dans l'eau. Ils sont en général caractérisés par le fait qu'ils comprennent, distribués de façon aléatoire :

a) de 90 à 99,9% en poids de motifs de formule générale (1) suivante :

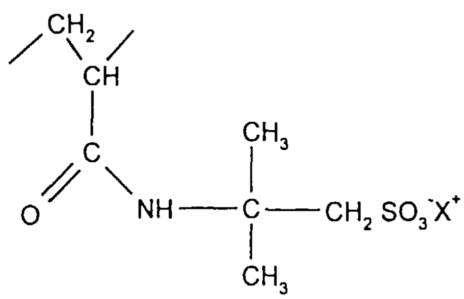

(1)

dans laquelle $X^+$ désigne un cation ou un mélange de cations, au plus 10% mol des cations $X^+$ pouvant être des protons $H^+$ ;

b) de 0,01 à 10% en poids de motifs réticulants provenant d'au moins un monomère ayant au moins deux doubles-liaisons oléfiniques ; les proportions en poids étant définis par rapport au poids total du polymère.

De façon préférentielle, les polymères de l'invention comportent un nombre de motifs de formule (1) dans une quantité suffisamment élevée pour obtenir un volume hydrodynamique du polymère en solution d'eau ayant un rayon allant de 10 à 500 nm, de distribution homogène et unimodale.

Les polymères selon l'invention plus particulièrement préférés comprennent de 98 à 99,5 % en poids de motifs de formule (1) et de 0,2 à 2 % en poids de motifs réticulants.

$X^+$ représente un cation ou un mélange de cations choisis en particulier parmi un proton, un cation de métal alcalin, un cation équivalent de celui d'un métal alcalino-terreux ou l'ion ammonium.

Plus particulièrement, 90 à 100% mole des cations sont des cations $NH_4^+$ et 0 à 10% mole sont des protons ($H^+$).

Les monomères de réticulation ayant au moins deux doubles-liaisons oléfiniques sont choisis par exemple parmi le dipropylèneglycol-diallyléther, les polyglycol-diallyléthers, le triéthylèneglycol-divinyléther, l'hydroquinone-diallyl-éther, le tétrallyl-oxéthanoyle ou d'autres allyl ou vinyléthers alcools polyfonctionnels, le diacrylate de tétraéthylèneglycol, la triallylamine, le triméthylolpropane-diallyléther, le méthylen-bis-acrylamide ou le divinylbenzène.

Les monomères de réticulation ayant au moins deux doubles-liaisons oléfiniques sont plus particulièrement choisis parmi ceux répondant à la formule générale (2) suivante :

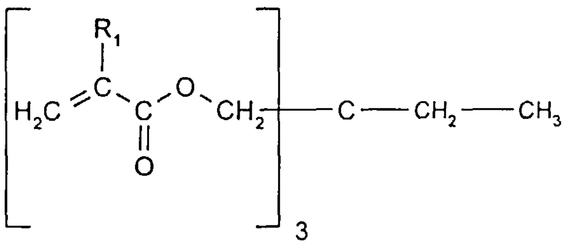 (2)

dans laquelle $R_1$ désigne un atome d'hydrogène ou un alkyle en $C_1$-$C_4$ et plus particulièrement méthyle (triméthylol propane triacrylate).

La réaction de polymérisation des polymères de l'invention produit non seulement des chaînes linéaires mais aussi des molécules de polymère ramifiées ou réticulées. Ces molécules peuvent être caractérisées notamment par leur comportement rhéologique dans l'eau mais plus particulièrement par la diffusion de la lumière dynamique.

Dans le cas de la caractérisation des molécules par la diffusion de la lumière dynamique, on mesure la distribution du volume hydrodynamique des structures du polymère. Les macromolécules dissoutes dans l'eau sont flexibles et entourées par une enveloppe de solvatation formée de molécules d'eau. Avec des polymères chargés comme ceux de l'invention, la taille des molécules dépend de la quantité de sel dans l'eau. Dans les solvants polaires, la charge uniforme le long de la chaîne principale du polymère conduit à une expansion importante de la chaîne polymérique. Le fait d'accroître la quantité de sel augmente la quantité d'électrolyte dans le solvant et écrante les charges uniformes du polymère. En plus des molécules transportées dans l'enveloppe de solvatation, les molécules de solvant sont fixées dans les cavités du polymère. Dans ce cas, les molécules de solvant font partie des macromolécules en solution et se déplacent à la même vitesse moyenne. Ainsi, le volume hydrodynamique décrit la dimension linéaire de la macromolécule et de ces molécules de solvatation.

Le volume hydrodynamique $v_h$ est déterminé par la formule suivante :

$$v_h = M/N_A \times (V_2 + dV_1)$$

avec:

- M désignant la masse en grammes de la macromolécule non-dissoute ;
- $N_A$ désignant le nombre d'Avogadro ;
- $V_1$ désignant le volume spécifique du solvant ;
- $V_2$ désignant le volume spécifique de la macromolécule ;
- d la masse en grammes du solvant qui est associé avec 1 gramme de macromolécule non-dissoute.

Si la particule hydrodynamique est sphérique, il est alors facile de calculer à partir du volume hydrodynamique le rayon hydrodynamique par la formule :

$$v_h = 4\Pi R^3/3$$

avec R désignant le rayon dynamique.

Les cas où les particules hydrodynamiques sont des sphères parfaites sont extrêmement rares. La majorité des polymères synthétiques impliquent des structures compactées ou des ellipsoides à haute excentricité. Dans ce cas, la détermination du rayon s'effectue sur une sphère qui est équivalente d'un point de vue frottement à la forme de la particule considérée.

En règle générale, on travaille sur des distributions de poids moléculaire et donc sur des distributions de rayon et de volume hydrodynamique. Pour les systèmes polydispersés, on doit calculer la distribution des coefficients de diffusion. De cette distribution , on en déduit les résultats relatifs à la distribution radiale et à la distribution des volumes hydrodynamiques.

Les volumes hydrodynamiques des polymères de l'invention sont en particulier déterminés par diffusion de la lumière dynamique à partir des coefficients de diffusion D selon STOKES-EINSTEIN de formule : $D = kT/6\Pi\eta R$ où k est la constante de Boltzmann, T la température absolue en degrés Kelvin, $\eta$ est la viscosité du solvant (eau) et R est le rayon hydrodynamique.

Ces coefficients de diffusion D sont mesurés selon la méthode de caractérisation d'un mélange de polymères

par diffusion au LASER décrite dans les références suivantes :

(1) Pecora, R ; Dynamic Light Scattering ; Plenium Press, New York, 1976 ;
(2) Chu, B ; Dynamic Light Scattering ; Academic Press, New York, 1994 ;
(3) Schmitz, KS ; Introduction to Dynamic Light Scattering ; Academic Press, New York, 1990;
(4) Provincher S.W.; Comp. Phys., 27, 213, 1982 ;
(5) Provincher S.W.; Comp. Phys., 27, 229, 1982 ;
(6) ALV Laservertriebgesellschaft mbH, Robert Bosch Str. 47, D-63225 Langen, Germany;
(7) ELS-Reinheimer Strasse 11, D-64846 Gross-Zimmern, Germany ;
(8) CHI WU et al, Macromolecules, 1995, 28,4914-4919.

Les polymères particulièrement préférés sont ceux présentant une viscosité mesurée au viscosimètre BROOKFIELD dans une solution d'eau à 2% et à 25 °C supérieure ou égale à 1000 mPa.s (ou cps), et plus préférentiellement allant de 5000 à 40000 mPa.s (ou cps) et plus particulièrement de 6500 à 35000 mPa.s (ou cps).

Les poly(acide 2-acrylamido 2-méthylpropane sulfonique) réticulés de l'invention peuvent être obtenus selon le procédé de préparation comprenant les étapes suivantes :

(a) on disperse ou on dissout le monomère acide 2-acrylamido 2-méthylpropane sulfonique sous forme libre dans une solution de tertio-butanol ou d'eau et de tertio-butanol ;

(b) on neutralise la solution ou la dispersion de monomère AMPS obtenue en (a) par une ou plusieurs bases minérales ou organiques, de préférence l'ammoniaque $NH_3$, dans une quantité permettant d'obtenir un taux de neutralisation des fonctions acides sulfoniques du polymère allant de 90 à 100% ;

(c) on ajoute à la solution ou dispersion obtenue en (b) le ou les monomères réticulants ;

(d) on effectue une polymérisation radicalaire classique en la présence d'amorceurs de radicaux libres à une température allant de 10 à 150°C ; le polymère précipitant dans la solution ou la dispersion à base de tertio-butanol.

Les poly(acide 2-acrylamido 2-méthylpropane sulfonique) réticulés, pratiquement ou totalement neutralisés sont présents dans les compositions cosmétiques ou dermatologiques de l'invention dans des concentrations allant préférentiellement de 0,01 à 20% en poids par rapport au poids total de la composition et plus préférentiellement de 0,1 à 10% en poids.

Les compositions selon l'invention peuvent contenir des actifs organiques acides cosmétiques et /ou dermatologiques, solubilisés et stabilisés dans le milieu aqueux acide en présence d'un ou plusieurs poly(acide 2-acrylamido 2-méthylpropane sulfonique) réticulés tels que définis ci-dessus.

Comme actifs acides organiques solubilisables dans la composition de l'invention, on peut citer l'acide ascorbique, l'acide kojique, l'acide citrique, l'acide caféique, l'acide salicylique et ses dérivés (par exemple acide n-octanoyl-5 ou décanoyl-5-salicylique), les α-hydroxyacides tels que l'acide lactique, l'acide méthyllactique, l'acide glucuronique, l'acide glycolique, l'acide pyruvique, l'acide 2-hydroxy-butanoïque, l'acide 2-hydroxypentanoïque, l'acide 2-hydroxyhexanoïque, l'acide 2-hydroxyheptanoïque, l'acide 2-hydroxyoctanoïque, l'acide 2-hydroxy-nonanoïque, l'acide 2-hydroxydécanoïque, l'acide 2-hydroxyundécanoïque, l'acide 2-hydroxydodécanoïque, l'acide 2-hydroxytétradécanoïque, l'acide 2-hydroxy-hexadécanoïque, l'acide 2-hydroxyoctadécanoïque, l'acide 2-hydroxytétracosanoïque, l'acide 2-hydroxyeïcosanoïque, l'acide mandélique, l'acide benzoïque, l'acide phényllactique, l'acide gluconique, l'acide galacturonique, l'acide citrique, l'acide aleuritique, l'acide ribonique, l'acide tartronique, l'acide tartrique, l'acide malique, l'acide fumarique, l'acide rétinoïque et ses dérivés, l'acide benzène 1,4-di(3-méthylidène 10-camphosulfonique), l'acide urocanique, l'acide 2-phényl benzimidazole 5-sulphonique, l'acide α-(oxo-2-bornylidène-3) toluène-4-sulfonique, l'acide 2-hydroxy-4-méthoxy-5-sulfonique. On peut aussi, utiliser tous les composés naturels ou synthétiques contenant de tels acides, comme les extraits végétaux et plus spécialement les extraits de fruits. On peut aussi solubiliser les dérivés xanthiques acides (caféine, théophylline), l'acide β-glycyrrhétinique, l'acide asiatique.

Les compositions de l'invention contiennent un milieu aqueux cosmétiquement acceptable, c'est-à-dire un milieu compatible avec toutes les matières kératiniques telles que la peau, les ongles, les muqueuses et les cheveux ou toute autre zone cutanée du corps.

Le milieu cosmétiquement et/ou dermatologiquement acceptable des compositions selon l'invention est plus particulièrement constitué d'eau et éventuellement de solvants organiques cosmétiquement et/ou dermatologiquement acceptables.

Les solvants organiques peuvent représenter de 5 % à 98 % du poids total de la composition. Ils peuvent être choisis dans le groupe constitué par les solvants organiques hydrophiles, les solvants organiques lipophiles, les

solvants amphiphiles ou leurs mélanges.

Parmi les solvants organiques aqueux, on peut citer par exemple des mono-alcools inférieurs linéaires ou ramifiés ayant de 1 à 8 atomes de carbone comme l'éthanol, le propanol, le butanol, l'isopropanol, l'isobutanol ; des polyéthylène glycols ayant de 6 à 80 oxydes d'éthylène ; des polyols tels que le propylène glycol, l'isoprène glycol, le butylène glycol, le glycérol, le sorbitol ; les mono- ou di-alkyle d'isosorbide dont les groupements alkyle ont de 1 à 5 atomes de carbone comme le diméthyl isosorbide ; les éthers de glycol comme le diéthylène glycol mono-méthyl ou mono-éthyl éther et les éthers de propylène glycol comme le dipropylène glycol méthyl éther.

Comme solvants organiques amphiphiles, on peut citer des polyols tels des dérivés de polypropylène glycol (PPG) tels que les esters de polypropylène glycol et d'acide gras, de PPG et d'alcool gras comme le PPG-23 oléyl éther et le PPG-36 oléate.

Comme solvants organiques lipophiles, on peut citer par exemple les esters gras tels que l'adipate de diisopropyle, l'adipate de dioctyle, les benzoates d'alkyle.

Afin que les compositions cosmétiques ou dermatologiques de l'invention soient plus agréables à utiliser (plus douce à l'application, plus nourrissante, plus émolliente), il est possible d'ajouter une phase grasse dans le milieu de ces composition.

La phase grasse représente de préférence, de 0 % à 50 % du poids total de la composition.

Cette phase grasse peut comporter une ou plusieurs huiles choisies de préférence dans le groupe constitué par:

- les silicones volatiles ou non-volatiles, linéaires, ramifiées ou cycliques, organomodifiées ou non, hydrosolubles ou liposolubles,
- les huiles minérales telles que l'huile de paraffine et de vaseline,
- les huiles d'origine animale telles que le perhydrosqualène,
- les huiles d'origine végétale telles que l'huile d'amande douce, l'huile d'avocat, l'huile de ricin, l'huile d'olive, l'huile de jojoba, l'huile de sésame, l'huile d'arachide, l'huile de macadamia, l'huile de pépins de raisin, l'huile de colza, l'huile de coprah,
- les huiles synthétiques telles que l'huile de purcellin, les isoparaffines,
- les huiles fluorées et perfluorées,
- les esters d'acides gras tels que l'huile de Purcellin.

Elle peut aussi comporter comme matière grasse un(e) ou plusieurs alcool gras, acides gras (acide stéarique) ou cires (paraffine, cires de polyéthylène, carnauba, cire d'abeilles).

De façon connue, toutes les compositions de l'invention peuvent contenir des adjuvants habituels dans les domaines cosmétique et dermatologique, d'autres gélifiants et/ou épaississants classiques aqueux ou lipophiles ; des actifs hydrophiles ou lipophiles ; des conservateurs ;des antioxydants ; des parfums ; des émulsionnants ; des agents hydratants ; des agents pigmentants ; des dépigmentants ; des agents kératolytiques ; des vitamines ; des émollients ; des séquestrants ; des tensio-actifs ; des polymères ; des agents alcanisants ou acidifiants ; des charges ; des agents anti-radicaux libres ; des céramides ; des filtres solaires (notamment ultraviolets), des répulsifs pour insectes; des agents amincissants. ; des matières colorantes ; des bactéricides ; des antipelliculaires. Les quantités de ces différents adjuvants sont celles classiquement utilisées dans les domaines considérés.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention peuvent se présenter sous toutes les formes appropriées pour une application topique, notamment sous forme de solutions du type lotion ou sérum, sous forme de gels aqueux, sous forme d'émulsions obtenues par dispersion d'une phase grasse dans une phase aqueuse (H/E) ou inversement (E/H), de consistance liquide, semi-liquide telles que des laits, des crèmes plus ou moins onctueuses, des pâtes. Ces compositions sont préparées selon les méthodes usuelles.

Les compositions selon l'invention peuvent être utilisées comme produits rincés ou comme produits non-rincés capillaires notamment pour le lavage, le soin, le conditionnement, le maintien de la coiffure ou la mise en forme des fibres kératiniques telles que les cheveux.

Elles peuvent être des produits de coiffage tels que des lotions de mise en plis, des lotions pour le brushing,

des compositions de fixation et de coiffage. Les lotions peuvent être conditionnées sous diverses formes notamment dans des vaporisateurs, des flacons pompes ou dans des récipients aérosols afin d'assurer une application de la composition sous forme vaporisée ou sous forme de mousse. De telles formes de conditionnement sont indiquées, par exemple, lorsqu'on souhaite obtenir un spray, une mousse pour la fixation ou le traitement des cheveux.

Les compositions de l'invention peuvent être également des shampooings, des compositions à rincer ou non, à appliquer avant ou après un shampooing, une coloration, une décoloration, une permanente ou un défrisage.

Les compositions de l'invention peuvent être également utilisées comme produit de soin et/ou l'hygiène tels que des crèmes de protection, de traitement ou de soin pour le visage, pour les mains ou pour le corps, des laits corporels de protection ou de soin, des lotions, gels ou mousses pour le soin de la peau et des muqueuses ou pour le nettoyage de la peau.

Les compositions de l'invention peuvent être également utilisées comme produit antisolaire.

Les compositions peuvent également consister en des préparations solides constituant des savons ou des pains de nettoyage.

Les compositions de l'invention peuvent être également utilisées comme produit de soin bucco-dentaire tel que des pâtes dentifrices.

Les compositions peuvent être des produits pour le maquillage.

Un autre objet de l'invention est un procédé de traitement non-thérapeutique cosmétique de la peau, du cuir chevelu, des cheveux, des cils, des sourcils, des ongles ou des muqueuses, caractérisé par le fait qu'on applique sur le support une composition telle que définie ci-dessus, selon la technique d'utilisation habituelle de cette composition. Par exemple : application de crèmes, de gels, de sérums, de lotions, de laits sur la peau, le cuir chevelu et/ou les muqueuses. Le type de traitement est fonction du ou des actifs acides solubilisés dans la composition.

Plus spécialement, l'invention se rapporte à un procédé non-thérapeutique pour dépigmenter la peau du visage et/ou du corps humain, consistant à appliquer sur la peau une composition telle définie ci-dessus.

L'invention a encore pour objet une utilisation de la composition ci-dessus pour préparer une pommade ou un onguent destiné à traiter thérapeutiquement le visage et/ou le corps humain, y compris les mains notamment pour traiter l'acné, les comédons chez les peaux grasses.

L'invention a encore pour objet une utilisation d'un polymère tel que défini ci-dessus, pour gélifier et/ou épaissir une composition cosmétique et/ou dermatologique contenant un milieu aqueux acide.

L'invention a encore pour objet une utilisation d'un polymère tel que défini ci-dessus, pour gélifier et/ou épaissir une composition cosmétique et/ou dermatologique contenant un milieu aqueux riche en solvant organique.

L'invention a encore pour objet une utilisation d'un polymère tel que défini ci-dessus, pour solubiliser et stabiliser un actif acide organique dans une composition cosmétique et/ou dermatologique contenant un milieu aqueux acide.

L'invention a encore pour objet une utilisation d'un polymère tel que défini ci-dessus, pour solubiliser et stabiliser un actif acide organique dans une composition cosmétique et/ou dermatologique contenant un milieu aqueux riche en solvant organique.

Les exemples suivants illustrent l'invention sans présenter un caractère limitatif.

## EXEMPLE DE PREPARATION A

Dans un ballon de 5 litres muni d'un agitateur, d'un réfrigérant à reflux, d'un thermomètre et d'un dispositif de conduite pour l'azote et pour l'ammoniaque, on introduit 2006,2 g de tertio-butanol puis 340,0 g d'acide 2-acrylamido 2-méthylpropane sulfonique que l'on disperse dans la solution sous forte agitation. Après 30 minutes, on ajoute l'ammoniaque par le conduit supérieur du ballon et on maintient le mélange réactionnel pendant 30 minutes à température ambiante jusqu'à l'obtention d'un pH de l'ordre de 6-6,5. On introduit ensuite 32,0 g d'une solution de triméthylolpropane triacrylate à 25% dans le tertio-butanol et on chauffe jusqu'à 60°C tandis que le milieu réactionnel est simultanément rendu inerte par apport de l'azote dans le ballon. Une fois cette température atteinte, on ajoute du dilauroylperoxyde. La réaction se déclenche aussitôt, ce qui se traduit par une montée de température et par une précipitation du polymérisat. 15 minutes après le début de la polymérisation, on introduit un courant d'azote. 30 minutes après l'ajout de l'amorceur, la température du milieu réactionnel atteint un maximum de 65-70°C. 30 minutes après avoir atteint cette température, on chauffe au reflux et on maintient dans ces conditions pendant 2 heures. On observe au cours de la réaction la formation d'une pâte épaisse. On refroidit jusqu'à la température ambiante et on filtre le produit obtenu. La pâte récupérée est ensuite séchée sous vide à 60-70°C pendant 24 heures. On obtient 391 g de poly(acide 2-acrylamido 2-méthylpropane sulfonique) réticulé et neutralisé, ayant viscosité mesurée au viscosimètre BROOKFIELD, mobile 4, à une vitesse de rotation de 100 tours/minutes dans une solution d'eau à 2 % et à 25 °C allant de 15000 à 35.000 mPa.s (ou cps). La viscosité du polymère sera choisie et contrôlée selon des moyens classiques en fonction de l'application cosmétique envisagée.

Le rayon hydrodynamique du polymère obtenu dans une solution aqueuse déterminé par diffusion de la lumière dynamique est de 440 nm.

**EXEMPLE DE PREPARATION B**

Dans un ballon de 5 litres muni d'un agitateur, d'un réfrigérant à reflux, d'un thermomètre et d'un dispositif de conduite pour l'azote et pour l'ammoniaque, on introduit 2006,2 g de tertio-butanol puis 340,0 g d'acide 2-acrylamido 2-méthylpropane sulfonique que l'on disperse dans la solution sous forte agitation. Après 30 minutes, on ajoute l'ammoniaque par le conduit supérieur du ballon et on maintient le mélange réactionnel pendant 30 minutes à température ambiante jusqu'à l'obtention d'un pH de l'ordre de 6-6,5. On introduit ensuite 19,2 g d'une solution de triméthylolpropane triacrylate à 25% dans le tertio-butanol et on chauffe jusqu'à 60°C tandis que le milieu réactionnel est simultanément rendu inerte par apport de l'azote dans le ballon. Une fois cette température atteinte, on ajoute du dilauroylperoxyde. La réaction se déclenche aussitôt, ce qui se traduit par une montée de température et par une précipitation du polymérisat. 15 minutes après le début de la polymérisation, on introduit un courant d'azote. 30 minutes après l'ajout de l'amorceur, la température du milieu réactionnel atteint un maximum de 65-70°C. 30 minutes après avoir atteint cette température, on chauffe au reflux et on maintient dans ces conditions pendant 2 heures. On observe au cours de la réaction la formation d'une pâte épaisse. On refroidit jusqu'à la température ambiante et on filtre le produit obtenu. La pâte récupérée est ensuite séchée sous vide à 60-70°C pendant 24 heures. On obtient 391 g de poly(acide 2-acrylamido 2-méthylpropane sulfonique) réticulé et neutralisé, ayant une viscosité mesurée au viscosimètre BROOKFIELD, mobile 4, à une vitesse de rotation de 100 tours/minutes dans une solution d'eau à 2 % et à 25 °C de l'ordre de 7000 mPa.s (ou cps).

Le rayon hydrodynamique du polymère obtenu dans une solution aqueuse déterminé par diffusion de la lumière dynamique est de 160 nm.

**EXEMPLE 1**: **Shampooing**

- Lauryl éther sulfate de Sodium vendu sous le nom d'EMPICOL ESB3/FL par la Société ALBRIGHT ET WILSON 10 g MA
- Poly(acide 2-acrylamido 2-méthylpropane sulfonique) réticulé et neutralisé par de l'ammoniaque, préparé selon le procédé de l'exemple de préparation B de viscosité de l'ordre de 7.000 mPa.s dans une solution d'eau à 2 % et à 25° C 1,5 g MA
- Acide citrique 3 g
- Eau pH ajusté à 4,8 (NaOH) qsp 100 g

Ce shampooing se présente sous l'aspect d'un liquide translucide, épaissi, stable et homogène. Il possède de bonnes propriétés moussantes.

**EXEMPLE 2** : **Gel transparent solaire**

- Glycérol 4 g
- Poly(acide 2-acrylamido 2-méthylpropane sulfonique) réticulé et neutralisé par de l'ammoniaque, préparé selon le procédé de l'exemple de préparation A de viscosité de l'ordre de 16.000 mPa.s dans une solution d'eau à 2% et à 25°C 1,0 g MA
- Acide benzène-1,4-di(3-méthylidène-10-camphosulfonique)] en solution aqueuse à 33% 6 g
- Propylèneglycol 18 g
- Eau déminéralisée stérilisée 70 g
  pH = 1,7

On obtient un gel stable, épais, transparent, onctueux et homogène.

**EXEMPLE 3** : **Gel transparent anti-moustiques**

- Glycérol 4 g

- Poly(acide 2-acrylamido 2-méthylpropane sulfonique) réticulé et neutralisé par de l'ammoniaque, préparé selon le procédé de l'exemple de préparation A de viscosité de l'ordre de 16.000 mPa.s dans une solution d'eau à 2% et à 25°C 0,8 g MA
- N-butyl, N-acétyl aminopropionate d'éthyle 15 g
- N,N diéthyl-M- toluamide 20 g
- Propylèneglycol 18 g
- Ethanol à 96° 23 g
- Eau déminéralisée stérilisée 19,2 g

pH = 3,95

On obtient un gel stable, épais, transparent, onctueux et homogène.

**<u>EXEMPLE 4</u> : Gel anti-age**

- Poly(acide 2-acrylamido 2-méthylpropane sulfonique) réticulé et neutralisé par de l'ammoniaque , préparé selon le procédé de l'exemple de préparation B de viscosité de l'ordre de 7.000 mPa.s dans une solution d'eau à 2% et à 25°C 2,0 g MA
- Acide benzène1,4-di(3-méthylidène 10-camphosulfonique) 0,7 g
- Acide lactique 2 g
- Glycérine 3 g
- Conservateur qs
- Eau distillée qsp 100 g

pH = 3,5

On obtient un gel stable, épais, transparent, onctueux et homogène.

**<u>EXEMPLE 5</u> : Gel dépigmentant**

- Poly(acide 2-acrylamido 2-méthylpropane sulfonique) réticulé et neutralisé par de l'ammoniaque, préparé selon le procédé de l'exemple de préparation B de viscosité de l'ordre de 7.000 mPa.s dans une solution d'eau à 2% et à 25°C 2,0 g MA
- Acide benzène1,4-di(3-méthylidène 10-camphosulfonique) 0,7 g
- Acide kojique 1 g
- Diméthicone copolyol 2 g
- Conservateur qs
- Eau distillée qsp 100 g

pH = 4,5

On obtient un gel stable, moyennement épais, transparent et homogène.

**<u>EXEMPLE 6</u> : Gel kératolytique**

- Poly(acide 2-acrylamido 2-méthylpropane sulfonique) réticulé et neutralisé par de l'ammoniaque , préparé selon le procédé de l'exemple de préparation B de viscosité de l'ordre de 7.000 mPa.s dans une solution d'eau à 2% et à 25°C 2,0 g MA
- Acide glycolique 2 g
- Diméthicone copolyol 2 g
- Conservateur qs
- Eau distillée qsp 100 g

pH = 2

On obtient un gel stable, transparent, lisse et homogène.

**EXEMPLE 7: Gel rafraichissant hydratant**

- Poly(acide 2-acrylamido 2-méthylpropane sulfonique) réticulé et neutralisé par de l'ammoniaque , préparé selon le procédé de l'exemple de préparation B de viscosité de l'ordre de 7.000 mPa.s dans une solution d'eau à 2% et à 25°C 2,0 g MA
- Alcool éthylique à 96° 20 g
- Glycérine 3 g
- Eau distillée qsp 100 g

pH = 4,8

On obtient un gel stable, transparent et homogène.

**EXEMPLE 8: Crème dépigmentante (émulsion huile-dans-eau)**

*Phase grasse*

- Glycéryl stéarate et PEG-100 stéarate 1,2 g
- PEG-20 stéarate 1,2 g
- Acide stéarique 0,6 g
- Alcool cétylique 1,2 g
- Octanoate de cétéaryle et myristate d'isopropyle 3 g
- Acide capryloyl salicylique 1,5 g
- Cyclométhicone 7 g
- PPG-3 myrystyl éther 7,5 g

*Phase aqueuse*

- Poly(acide 2-acrylamido 2-méthylpropane sulfonique) réticulé et neutralisé par de l'ammoniaque, préparé selon le procédé de l'exemple de préparation B de viscosité de l'ordre de 7.000 mPa.s dans une solution d'eau à 2% et à 25°C 1,2 g MA
- Acide kojique 1 g
- Acide caféïque 0,2 g
- PEG-8 15 g
- Conservateur qs
- Eau déminéralisée stérilisée qsp 100 g

pH 3,1

On obtient une crème lisse, blanche et brillante.

## Revendications

1. Composition cosmétique et/ou dermatologique contenant un milieu aqueux acide ayant un pH inférieur à 5, et au moins un poly(acide 2-acrylamido 2-méthylpropane sulfonique) réticulé neutralisé à au moins 90%.

2. Composition selon la revendication 1, caractérisée en ce que le pH du milieu aqueux varie de 1 à 4.

3. Composition selon la revendication 1 ou 2, caractérisée par le fait que le poly(acide 2-acrylamido 2-méthylpropane sulfonique) réticulé et neutralisé à au moins 90% comprend, distribués de façon aléatoire :

   a) de 90 à 99,9% en poids de motifs de formule générale (1) suivante :

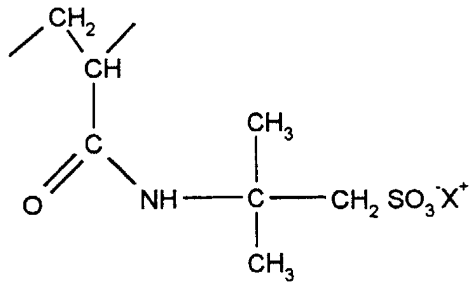

(1)

dans laquelle $X^+$ désigne un cation ou un mélange de cations, au plus 10% mol des cations $X^+$ pouvant être des protons $H^+$ ;

b) de 0,01 à 10% en poids de motifs réticulants provenant d'au moins un monomère ayant au moins deux doubles liaisons oléfiniques ; les proportions en poids étant définis par rapport au poids total du polymère.

**4.** Composition selon l'une quelconque des revendications 1 à 3, caractérisée par le fait que le poly(acide 2-acrylamido 2-méthylpropane sulfonique) réticulé comporte un nombre de motifs de formule (1) dans une quantité suffisamment élevée pour obtenir un volume hydrodynamique du polymère en solution d'eau ayant un rayon allant de 10 à 500 nm , de distribution homogène et unimodale.

**5.** Composition selon l'une quelconque des revendications 1 à 4, caractérisée par le fait que le poly(acide 2-acrylamido 2-méthylpropane sulfonique) réticulé comporte de 98 à 99,5 % en poids de motifs de formule (1) et de 0,2 à 2 % en poids de motifs réticulants.

**6.** Composition selon l'une quelconque des revendications 1 à 5, caractérisée par le fait que dans la formule (1) le cation $X^+$ est $NH_4^+$.

**7.** Composition selon l'une quelconque des revendications 1 à 6, caractérisée par le fait que les monomères réticulants répondent à la formule générale (2) suivante :

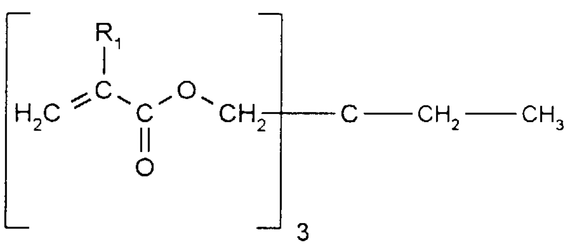

(2)

dans laquelle $R_1$ désigne un atome d'hydrogène ou un alkyle en $C_1$-$C_4$.

**8.** Composition selon l'une quelconque des revendications 1 à 7, caractérisée par le fait que le poly(acide 2-acrylamido 2-méthylpropane sulfonique) est réticulé par le triméthylol propane triacrylate.

**9.** Composition selon l'une quelconque des revendications 1 à 8, caractérisée par le fait que le poly(acide 2-acrylamido 2-méthylpropane sulfonique) réticulé présente une viscosité mesurée au viscosimètre BROOKFIELD, mobile 4, à une vitesse de rotation de 100 tours/minutes, dans une solution d'eau à 2 % et à 25 °C, supérieure ou égale à 1000 mPa.s.

**10.** Composition selon l'une quelconque des revendications 1 à 9, caractérisée par le fait que le poly(acide 2-acrylamido 2-méthylpropane sulfonique) réticulé présente une viscosité mesurée au viscosimètre BROOKFIELD, mobile 4, à une vitesse de rotation de 100 tours/minutes, dans une solution d'eau à 2 % et à 25 °C allant de 5000 à 40000 mPa.s et plus particulièrement de 6500 à 35000 mPa.s.

**11.** Composition selon l'une quelconque des revendications 1 à 10, caractérisée par le fait que le poly(acide 2-acrylamido 2-méthylpropane sulfonique) réticulé est présent dans des concentrations allant préférentiellement de 0,01 à 20% en poids par rapport au poids total de la composition et plus préférentiellement de.0,1 à 10% en poids.

**12.** Composition selon l'une quelconque des revendications 1 à 11, caractérisée en ce que le milieu cosmétiquement et/ou dermatologiquement acceptable est constitué d'eau ou d'eau et d'au moins un solvant organique choisi dans le groupe constitué par les solvants organiques hydrophiles, les solvants organiques lipophiles, les solvants amphiphiles ou leurs mélanges.

**13.** Composition selon la revendication 12, caractérisée en ce que les solvants organiques sont choisis dans le groupe constitué par les alcools mono- ou polyfonctionnels, les polyéthylène glycols éventuellement oxyéthylénés, les esters de propylène glycol, le sorbitol et ses dérivés, les di alkyles d'isosorbide, les éthers de glycol et les éthers de propylène glycol, les esters gras.

**14.** Composition selon la revendication 12 ou 13, caractérisée en ce que le ou les solvants organiques représentent de 5 % à 98 % du poids total de la composition.

**15.** Composition selon l'une quelconque des revendications 1 à 14, caractérisée en ce qu'elle comprend en plus au moins une phase grasse.

**16.** Composition selon la revendication 15, caractérisée en ce que la phase grasse représente de 0 à 50% du poids total de la composition.

**17.** Composition selon l'une quelconque des revendications 1 à 16, caractérisée en ce qu'elle comprend en plus au moins un actif acide organique cosmétiquement et/ou dermatologiquement acceptable.

**18.** Composition selon la revendication 17, caractérisée en ce que l'actif acide organique est choisi dans le groupe constitué par l'acide ascorbique, l'acide kojique, l'acide citrique, l'acide caféique, l'acide salicylique et ses dérivés, les $\alpha$-hydroxyacides, l'acide mandélique, l'acide benzoïque, l'acide phényllactique, l'acide gluconique, l'acide galacturonique, l'acide aleuritique, l'acide ribonique, l'acide tartronique, l'acide tartrique, l'acide malique, l'acide fumarique, l'acide rétinoïque et ses dérivés, l'acide benzène 1,4-di(3-méthylidène 10-camphosulfonique), l'acide urocanique, l'acide 2-phényl benzimidazole 5-sulphonique, l'acide $\alpha$-(oxo-2-bornylidène-3) toluène-4-sulfonique, l'acide 2-hydroxy-4-méthoxy-5-sulfonique, les extraits végétaux contenant des acides et plus spécialement les extraits de fruits, les dérivés xanthiques acides, l'acide $\beta$-glycyrrhétinique, l'acide asiatique.

**19.** Composition selon l'une quelconque des revendications 1 à 18, caractérisée par le fait qu'elle contient en plus au moins un additif choisi dans le groupe constitué par les gélifiants et/ou épaississants classiques hydrophiles ou lipophiles ; des actifs hydrophiles ou lipophiles ; des conservateurs ;des antioxydants ; des parfums ; des émulsionnants ; des agents hydratants ; des agents pigmentants ; des dépigmentants ; des agents kératolytiques ; des vitamines ; des émollients ; des sequestrants ; des tensio-actifs ; des polymères ; des agents alcanisants ou acidifiants ; des charges ; des agents anti-radicaux libres ; des céramides ; des filtres solaires (notamment ultraviolets), des répulsifs pour insectes; des agents amincissants ; des matières colorantes ; des bactéricides; des antipelliculaires.

**20.** Composition selon l'une quelconque des revendications 1 à 19, caractérisée par le fait qu'elle est un produit capillaire rincé ou non-rincé pour le lavage, le soin, le conditionnement, le maintien de la coiffure ou la mise en forme des cheveux.

**21.** Composition selon l'une quelconque des revendications 1 à 19, caractérisée par le fait qu'elle est un produit de soin et/ou d'hygiène.

**22.** Composition selon l'une quelconque des revendications 1 à 19, caractérisée par le fait qu'elle est un produit de maquillage.

**23.** Composition selon l'une quelconque des revendications 1 à 19, caractérisée par le fait qu'elle est un produit antisolaire.

**24.** Composition selon l'une quelconque des revendications 1 à 19, caractérisée par le fait qu'elle est un produit de soin bucco-dentaire.

**25.** Procédé de traitement non thérapeutique cosmétique de la peau, du cuir chevelu, des cheveux, des cils, des sourcils, des ongles ou des muqueuses, caractérisé par le fait qu'on applique sur le support une composition telle que définie selon l'une quelconque des revendications 1 à 24.

**26.** Procédé selon la revendication 25, pour dépigmenter la peau du visage et/ou du corps humain consistant à appliquer sur la peau une composition selon l'une quelconque des revendications 1 à 19.

**27.** Utilisation de la composition selon l'une quelconque des revendications 1 à 19, pour préparer une pommade ou un onguent destiné à traiter thérapeutiquement le visage et/ou le corps humain.

**28.** Utilisation d'un polymère tel que défini dans l'une quelconque des revendications 1 à 10, pour gélifier et/ou épaissir une composition cosmétique et/ou dermatologique contenant un milieu aqueux acide ayant un pH inférieur à 5.

**29.** Utilisation d'un polymère tel que défini dans l'une quelconque des revendications 1 à 10, pour gélifier et/ou épaissir une composition cosmétique et/ou dermatologique contenant un milieu aqueux contenant au moins 45% en poids de solvant organique.

**30.** Utilisation d'un polymère tel que défini dans l'une quelconque des revendications 1 à 10, pour solubiliser et stabiliser un actif acide organique dans une composition cosmétique et/ou dermatologique contenant un milieu aqueux acide.

**31.** Utilisation selon la revendication 30, dans laquelle la composition contient un milieu aqueux acide contenant au moins 45% en poids de solvant organique.

## Patentansprüche

**1.** Kosmetische und/oder dermatologische Zusammensetzung, die ein saures, wässeriges Medium, das einen pH-Wert unter 5 aufweist, und mindestens eine vernetzte und zu mindestens 90 % neutralisierte Poly(2-acrylamido-2-methylpropan-sulfonsäure) enthält.

**2.** Zusammensetzung nach Anspruch 1, dadurch gekennzeichnet, daß der pH-Wert des wässerigen Mediums im Bereich von 1 bis 4 liegt.

**3.** Zusammensetzung nach Anspruch 1 oder 2, dadurch gekennzeichnet, daß die vernetzte und zu mindestens 90 % neutralisierte Poly(2-acrylamido-2-methylpropan-sulfonsäure) in statistischer Verteilung enthält:

a) 90 bis 99,9 Gew.-% Einheiten der folgenden allgemeinen Formel (1):

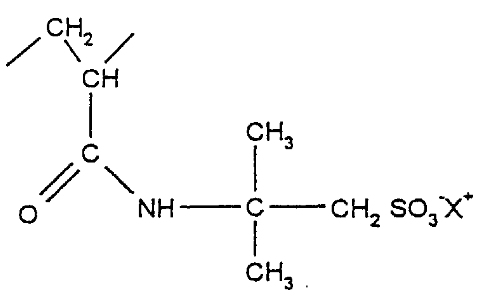

(1),

worin $X^+$ ein Kation oder ein Gemisch von Kationen bedeutet, wobei höchstens 10 Mol-% der Kationen $X^+$ Protonen ($H^+$) sein können,
und
b) 0,01 bis 10 Gew.-% vernetzende Einheiten, die von mindestens einem Monomer stammen, das mindestens zwei olefinische Doppelbindungen aufweist,

wobei die Gewichtsanteile bezogen auf das Gesamtgewicht des Polymers angegeben sind.

**4.** Zusammensetzung nach einem der Ansprüche 1 bis 3, dadurch gekennzeichnet, daß die vernetzte Poly(2-acrylamido-2-methylpropan-sulfonsäure) Einheiten der Formel (1) in einer Anzahl aufweist, die groß genug ist, um Polymerpartikel zu erhalten, deren hydrodynamisches Volumen in wässeriger Lösung einen Radius im Bereich von 10 bis 500 nm aufweist und deren Verteilung homogen und unimodal ist.

**5.** Zusammensetzung nach einem der Ansprüche 1 bis 4, dadurch gekennzeichnet, daß die vernetzte Poly(2-acrylamido-2-methylpropan-sulfonsäure) 98 bis 99,5 Gew.-% Einheiten der Formel (1) und 0,2 bis 2 Gew.-% vernetzende Einheiten aufweist.

**6.** Zusammensetzung nach einem der Ansprüche 1 bis 5, dadurch gekennzeichnet, daß in der Formel (1) das Kation $X^+$ $NH_4^+$ ist.

**7.** Zusammensetzung nach einem der Ansprüche 1 bis 6, dadurch gekennzeichnet, daß die vernetzenden Monomere der folgenden allgemeinen Formel (2) entsprechen:

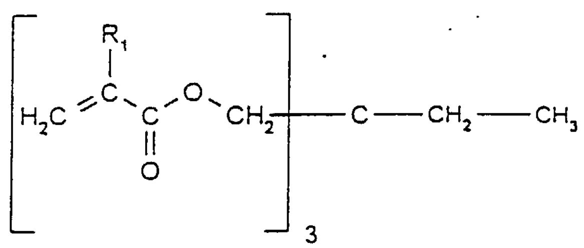

(2),

worin $R_1$ ein Wasserstoffatom oder eine $C_{1-4}$-Alkylgruppe bedeutet.

**8.** Zusammensetzung nach einem der Ansprüche 1 bis 7, dadurch gekennzeichnet, daß die Poly(2-acrylamido-2-methylpropan-sulfonsäure) mit Trimethylolpropantriacrylat vernetzt ist.

**9.** Zusammensetzung nach einem der Ansprüche 1 bis 8, dadurch gekennzeichnet, daß die vernetzte Poly(2-acrylamido-2-methylpropan-sulfonsäure) eine mit einem BROOKFIELD-Viskosimeter mit einem Rotationskörper 4 bei einer Rotationsgeschwindigkeit von 100 U/min in einer 2%igen wässerigen Lösung bei 25 °C bestimmte Viskosität von mindestens 1 000 mPa·s aufweist.

**10.** Zusammensetzung nach einem der Ansprüche 1 bis 9, dadurch gekennzeichnet, daß die vernetzte Poly(2-acrylamido-2-methylpropan-sulfonsäure) eine mit einem BROOKFIELD-Viskosimeter mit einem Rotationskörper 4 bei einer Rotationsgeschwindigkeit von 100 U/min in einer 2%igen wässerigen Lösung bei 25 °C bestimmte Viskosität von 5 000 bis 40 000 mPa·s und insbesondere von 6 500 bis 35 000 mPa·s aufweist.

**11.** Zusammensetzung nach einem der Ansprüche 1 bis 10, dadurch gekennzeichnet, daß die vernetzte Poly(2-acrylamido-2-methylpropan-sulfonsäure) in Konzentrationen vorliegt, die vorzugsweise im Bereich von 0,01 bis 20 Gew.-%, bezogen auf das Gesamtgewicht der Zusammensetzung, und noch bevorzugter im Bereich von 0,1 bis 10 Gew.-% liegen.

**12.** Zusammensetzung nach einem der Ansprüche 1 bis 11, dadurch gekennzeichnet, daß das kosmetisch und/oder dermatologisch akzeptable Medium aus Wasser oder aus Wasser und mindestens einem organischen Lösemittel besteht, das unter den hydrophilen organischen Lösemitteln, den lipophilen organischen Lösemitteln, den amphiphilen Lösemitteln oder deren Gemischen ausgewählt ist.

**13.** Zusammensetzung nach Anspruch 12, dadurch gekennzeichnet, daß die organischen Lösemittel unter den monofunktionellen oder polyfunktionellen Alkoholen, gegebenenfalls ethoxylierten Polyethylenglykolen, Propylenglykolestern, Sorbit und seinen Derivaten, Dialkylisosorbiden, Glykolethern, Propylenglykolethern und Fettestern ausgewählt sind.

**14.** Zusammensetzung nach Anspruch 12 oder 13, dadurch gekennzeichnet, daß das organische Lösemittel oder die organischen Lösemittel 5 % bis 98 % des Gesamtgewichts der Zusammensetzung ausmachen.

**15.** Zusammensetzung nach einem der Ansprüche 1 bis 14, dadurch gekennzeichnet, daß sie ferner mindestens eine Fettphase enthält.

**16.** Zusammensetzung nach Anspruch 15, dadurch gekennzeichnet, daß die Fettphase 0 bis 50 % des Gesamtgewichts der Zusammensetzung ausmacht.

**17.** Zusammensetzung nach einem der Ansprüche 1 bis 16, dadurch gekennzeichnet, daß sie ferner mindestens einen kosmetisch und/oder dermatologisch akzeptablen sauren, organischen Wirkstoff enthält.

**18.** Zusammensetzung nach Anspruch 17, dadurch gekennzeichnet, daß der saure, organische Wirkstoff ausgewählt ist unter Ascorbinsäure, Kojisäure, Citronensäure, Kaffeesäure, Salicylsäure und ihren Derivaten, α-Hydroxysäuren, Mandelsäure, Benzoesäure, Phenylmilchsäure, Gluconsäure, Galacturonsäure, Aleuritinsäure, Ribonsäure, Tartronsäure, Weinsäure, Äpfelsäure, Fumarsäure, Retinoesäure und ihren Derivaten, Benzol-1,4-di(3-methylidencampher-10-sulfonsäure), Urocansäure, 2-Phenylbenzimidazol-5-sulfonsäure, α-(2-Oxo-3-bornyliden)-toluol-4-sulfonsäure, 2-Hydroxy-4-methoxy-5-sulfonsäure, Pflanzenextrakten, die Säuren enthalten, und spezieller Fruchtextrakten, sauren Xanthinderivaten, β-Glycyrrhetinsäure und Asiatsäure.

**19.** Zusammensetzung nach einem der Ansprüche 1 bis 18, dadurch gekennzeichnet, daß sie ferner mindestens einen Zusatzstoff enthält, der ausgewählt ist unter herkömmlichen wässerigen oder lipophilen Gelbildnern und/oder Verdickungsmitteln, hydrophilen oder lipophilen Wirkstoffen, Konservierungsmitteln, Antioxidantien, Parfums, Emulgatoren, Hydratisierungsmitteln, pigmentierenden Mitteln, depigmentierenden Mitteln, keratolytischen Mitteln, Vitaminen, Emollentien, Maskierungsmitteln, grenzflächenaktiven Stoffen, Polymeren, Mitteln zum Alkalischmachen oder zum Ansäuren, Füllstoffen, Mitteln gegen freie Radikale, Ceramiden, Sonnenschutzfiltern (insbesondere UV-Filtern), insektenabwehrenden Mitteln, schlankmachenden Mitteln, Färbemitteln, Bakteriziden und Mitteln gegen Schuppen.

**20.** Zusammensetzung nach einem der Ansprüche 1 bis 19, dadurch gekennzeichnet, daß es sich um ein Produkt zur Haarbehandlung, das ausgespült wird oder im Haar verbleibt, zum Waschen, zur Pflege, zum Konditionieren, für den Halt der Frisur oder für die Formgebung der Haare handelt.

**21.** Zusammensetzung nach einem der Ansprüche 1 bis 19, dadurch gekennzeichnet, daß sie ein Pflegeprodukt und/ oder ein Hygieneprodukt ist.

**22.** Zusammensetzung nach einem der Ansprüche 1 bis 19, dadurch gekennzeichnet, daß sie ein Schminkprodukt ist.

**23.** Zusammensetzung nach einem der Ansprüche 1 bis 19, dadurch gekennzeichnet, daß sie ein Sonnenschutzprodukt ist.

**24.** Zusammensetzung nach einem der Ansprüche 1 bis 19, dadurch gekennzeichnet, daß sie ein Produkt für die Mund- und Zahnpflege ist.

**25.** Verfahren zur nichttherapeutischen kosmetischen Behandlung der Haut, der Kopfhaut, der Haare, der Wimpern, der Augenbrauen, der Nägel oder der Schleimhäute, dadurch gekennzeichnet, daß eine in einem der Ansprüche 1 bis 24 definierte Zusammensetzung auf den Träger aufgebracht wird.

**26.** Verfahren nach Anspruch 25 zur Depigmentierung der Gesichtshaut und/oder der menschlichen Körperhaut, das darin besteht, eine Zusammensetzung nach einem der Ansprüche 1 bis 19 auf die Haut aufzutragen.

**27.** Verwendung der Zusammensetzung nach einem der Ansprüche 1 bis 19 zur Herstellung einer Pomade oder einer Salbe, die zur therapeutischen Behandlung des Gesichts und/ oder des menschlichen Körpers bestimmt ist.

**28.** Verwendung eines in einem der Ansprüche 1 bis 10 definierten Polymers zum Gelieren und/oder Verdicken einer kosmetischen und/oder dermatologischen Zusammensetzung, die ein saures, wässeriges Medium mit einem pH-Wert unter 5 enthält.

**29.** Verwendung eines in einem der Ansprüche 1 bis 10 definierten Polymers zum Gelieren und/oder Verdicken einer kosmetischen und/oder dermatologischen Zusammensetzung, die ein wässeriges Medium enthält, das mindestens 45 Gew.-% organisches Lösemittel enthält.

**30.** Verwendung eines in einem der Ansprüche 1 bis 10 definierten Polymers zum Solubilisieren und Stabilisieren eines sauren, organischen Wirkstoffs in einer kosmetischen und/oder dermatologischen Zusammensetzung, die ein saures, wässeriges Medium enthält.

**31.** Verwendung nach Anspruch 30, worin die Zusammensetzung ein saures, wässeriges Medium enthält, das mindestens 45 Gew.-% organisches Lösemittel enthält.

## Claims

**1.** Cosmetic and/or dermatological composition comprising an acidic aqueous medium having a pH of less than 5 and at least one crosslinked poly(2-acrylamido-2-methylpropanesulphonic acid) neutralized to at least 90%.

**2.** Composition according to Claim 1, characterized in that the pH of the aqueous medium varies from 1 to 4.

**3.** Composition according to Claim 1 or 2, characterized in that the crosslinked poly(2-acrylamido-2-methylpropanesulphonic acid) neutralized to at least 90% comprises, distributed randomly:

a) from 90 to 99.9% by weight of units of following general formula (1):

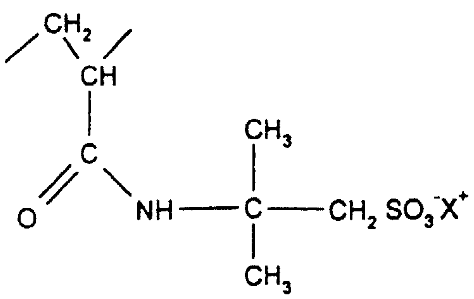

(1)

in which $X^+$ denotes a cation or a mixture of cations, it being possible for at most 10 mol % of the cations $X^+$ to be protons $H^+$;

b) from 0.01 to 10% by weight of crosslinking units originating from at least one monomer having at least two olefinic double bonds; the proportions by weight being defined with respect to the total weight of the polymer.

**4.** Composition according to any one of Claims 1 to 3, characterized in that the crosslinked poly(2-acrylamido-2-methylpropanesulphonic acid) comprises a number of units of formula (1) in an amount which is sufficiently high to obtain a hydrodynamic volume of the polymer, in solution in water, having a radius ranging from 10 to 500 nm, with a homogeneous and unimodal distribution.

**5.** Composition according to any one of Claims 1 to 4, characterized in that the crosslinked poly(2-acrylamido-2-methylpropanesulphonic acid) comprises from 98 to 99.5% by weight of units of formula (1) and from 0.2 to 2% by weight of crosslinking units.

**6.** Composition according to any one of Claims 1 to 5, characterized in that, in the formula (1), the cation $X^+$ is $NH_4^+$.

**7.** Composition according to any one of Claims 1 to 6, characterized in that the crosslinking monomers correspond to the following general formula (2):

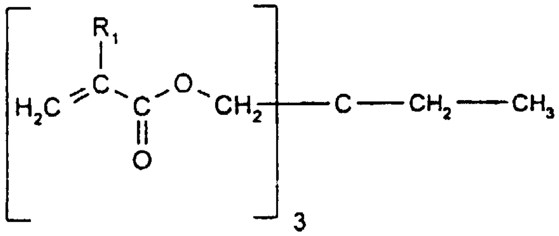

(2)

in which $R_1$ denotes a hydrogen atom or a $C_1$-$C_4$ alkyl.

**8.** Composition according to any one of Claims 1 to 7, characterized in that the poly(2-acrylamido-2-methylpropanesulphonic acid) is crosslinked with trimethylolpropane triacrylate.

**9.** Composition according to any one of Claims 1 to 8, characterized in that the crosslinked poly(2-acrylamido-2-methylpropanesulphonic acid) exhibits a viscosity, measured with a Brookfield viscometer, rotor 4, at a rotational speed of 100 revolutions/minute in a 2% solution in water at 25°C, of greater than or equal to 1000 mPa·s.

**10.** Composition according to any one of Claims 1 to 9, characterized in that the crosslinked poly(2-acrylamido-2-methylpropanesulphonic acid) exhibits a viscosity, measured with a Brookfield viscometer, rotor 4, at a rotational speed of 100 revolutions/minute in a 2% solution in water at 25°C, ranging from 5000 to 40,000 mPa·s and more particularly from 6500 to 35,000 mPa·s.

**11.** Composition according to any one of Claims 1 to 10, characterized in that the crosslinked poly(2-acrylamido-2-methylpropanesulphonic acid) is present in concentrations preferentially ranging from 0.01 to 20% by weight with respect to the total weight of the composition and more preferentially from 0.1 to 10% by weight.

**12.** Composition according to any one of Claims 1 to 11, characterized in that the cosmetically and/or dermatologically acceptable medium is composed of water or of water and of at least one organic solvent chosen from the group composed of hydrophilic organic solvents, lipophilic organic solvents, amphiphilic solvents or their mixtures.

**13.** Composition according to Claim 12, characterized in that the organic solvents are chosen from the group composed of mono- or polyfunctional alcohols, optionally oxyethylenated polyethylene glycols, propylene glycol esters, sorbitol and its derivatives, dialkyl isosorbides, glycol ethers and propylene glycol ethers, or fatty esters.

**14.** Composition according to Claim 12 or 13, characterized in that the organic solvent or solvents represent from 5% to 98% of the total weight of the composition.

**15.** Composition according to any one of Claims 1 to 14, characterized in that it additionally comprises at least one fatty phase.

**16.** Composition according to Claim 15, characterized in that the fatty phase represents from 0 to 50% of the total weight of the composition.

**17.** Composition according to any one of Claims 1 to 16, characterized in that it additionally comprises at least one cosmetically and/or dermatologically acceptable organic acid active principle.

**18.** Composition according to Claim 17, characterized in that the organic acid active principle is chosen from the group composed of ascorbic acid, kojic acid, citric acid, caffeic acid, salicylic acid and its derivatives, α-hydroxy acids, mandelic acid, benzoic acid, phenyllactic acid, gluconic acid, galacturonic acid, aleuritic acid, ribonic acid, tartronic acid, tartaric acid, malic acid, fumaric acid, retinoic acid and its derivatives, benzene-1,4-di (3-methylidene-10-camphorsulphonic acid), urocanic acid, 2-phenylbenzimidazole-5-sulphonic acid, α-(2-oxo-3-bornylidene)toluene-4-sulphonic acid, 2-hydroxy-4-methoxy-5-sulphonic acid, plant extracts comprising acids and more especially fruit extracts, acidic xanthine derivatives, β-glycyrrhetinic acid or asiatic acid.

**19.** Composition according to any one of Claims 1 to 18, characterized in that it additionally comprises at least one additive chosen from the group composed of conventional hydrophilic or lipophilic gelling and/or thickening agents;

hydrophilic or lipophilic active principles; preservatives; antioxidants; fragrances; emulsifiers; moisturizing agents; pigmenting agents; depigmenting agents; keratolytic agents; vitamins; emollients; sequestering agents; surfactants; polymers; basifying or acidifying agents; fillers; agents for combatting free radicals; ceramides; sunscreen agents (in particular ultraviolet screening agents); insect repellents; slimming agents; colouring materials; bactericides; or antidandruff agents.

**20.** Composition according to any one of Claims 1 to 19, characterized in that it is a rinse-out or leave-in hair product for washing, caring for, conditioning or form retention of the hairstyle or shaping the hair.

**21.** Composition according to any one of Claims 1 to 19, characterized in that it is a care and/or hygiene product.

**22.** Composition according to any one of Claims 1 to 19, characterized in that it is a make-up product.

**23.** Composition according to any one of Claims 1 to 19, characterized in that it is an anti-sun product.

**24.** Composition according to any one of Claims 1 to 19, characterized in that it is an oral care product.

**25.** Process for the non-therapeutic cosmetic treatment of the skin, scalp, hair, eyelashes, eyebrows, nails or mucous membranes, characterized in that a composition as defined according to any one of Claims 1 to 24 is applied on the substrate.

**26.** Process according to Claim 25 for depigmenting the skin of the human face and/or body which consists in applying a composition according to any one of Claims 1 to 19 on the skin.

**27.** Use of the composition according to any one of Claims 1 to 19 for preparing a pomade or an ointment intended for the therapeutic treatment of the human face and/or body.

**28.** Use of a polymer as defined in any one of Claims 1 to 10 for gelling and/or thickening a cosmetic and/or dermatological composition comprising an acidic aqueous medium having a pH of less than 5.

**29.** Use of a polymer as defined in any one of Claims 1 to 10 for gelling and/or thickening a cosmetic and/or dermatological composition comprising an aqueous medium comprising at least 45% by weight of organic solvent.

**30.** Use of a polymer as defined in any one of Claims 1 to 10 for dissolving and stabilizing an organic acid active principle in a cosmetic and/or dermatological composition comprising an acidic aqueous medium.

**31.** Use according to Claim 30, in which the composition comprises an acidic aqueous medium comprising at least 45% by weight of organic solvent.